# EUROPEAN PATENT SPECIFICATION

(11) **EP 2 144 653 B1**
(45) Date of publication and mention of the grant of the patent: **28.11.2018**
(21) Application number: 08744541.7
(22) Date of filing: 28.03.2008
(51) Int. Cl.: A61M 15/00, B65D 83/14

(54) **MANUFACTURE OF METERED DOSE VALVE COMPONENTS**
HERSTELLUNG VON BESTANDTEILEN EINES VENTILS FÜR ABGEMESSENE DOSEN
FABRICATION DE COMPOSANTS DE VALVE DOSEUSE

(30) Priority: 10.05.2007 US 917201 P
(43) Date of publication of application: 20.01.2010
(73) Proprietor: 3M Innovative Properties Company, St. Paul, MN 55133-3427 (US)
(72) Inventor: SIVIGNY, Michael B.,, Saint Paul, Minnesota 55133-3427 (US); HODSON, Peter D., Bracknell, Berkshire RG12 8HT (GB)
(74) Representative: Vossius & Partner Patentanwälte Rechtsanwälte mbB
(86) International application number: PCT/US2008/058571
(87) International publication number: WO 2008/140869

(56) References cited:
- EP-A1- 0 824 023
- WO-A1-02/43794
- DE-C- 880 279
- GB-A- 2 004 526
- GB-A- 2 198 117
- US-A- 4 597 512
- US-A- 5 593 069
- US-A1- 2004 050 970
- US-A1- 2006 118 107
- US-B1- 7 185 648

## Description

The present invention relates generally to the manufacture of valve stems for use in pressurized metered dose dispensing valves for dispensing pharmaceutical aerosol formulations as disclosed e.g. in document GB 2 198 117. The use of aerosols to administer medicament has been known for several decades. Such aerosol formulations generally comprise medicament, one or more propellants, (e.g. chlorofluorocarbons and more recently hydrogen-containing fluorocarbons, such as propellant 134a (CF₃CH₂F) and propellant 227 (CF₃CHFCF₃)) and, if desired, other excipients, such as a surfactant and/or a solvent, such as ethanol.
Pharmaceutical aerosols for inhalation, nasal, or sublingual administration generally comprise a container or vial of the aerosol formulation equipped with a metered dose dispensing valve. Although there are many different designs of metered dose valves, most comprise a metering chamber defined in part by a valve body and a valve stem that slides through a diaphragm into the metering chamber. When the valve is in its non-dispensing position, the diaphragm maintains a closed seal around the valve stem. The valve stem typically includes an opening (typically a side port) in communication with a discharge passageway inside the valve stem. When such a valve is actuated, the valve stem typically moves inwardly, so that the side port of the valve stem passes the diaphragm and enters into the metering chamber, allowing the contents of the metering chamber to pass through the side port and passageway, and to exit through the stem outlet.
Other metered dose dispensing valves such as those described in patent applications WO 2004/022142 and WO 2004/022143 form a transient metering chamber upon actuation. For example WO 2004/022142 describes *inter alia,* a metered dose valve comprising a valve body having an internal chamber with a valve stem positioned therein which has a body portion that is generally triangular through to diamond shaped in its vertical cross-section and a stem portion in sealing engagement with a diaphragm seal. Upon initial inward movement of the valve stem of such a valve, the inwardly facing surface of the valve stem body portion forms a face seal with a metering gasket provided on the valve body thus forming a transient metering chamber between *inter alia* the outwardly facing surface of the valve stem body portion and a portion of the valve body. Upon further movement of the valve stem inwardly, an opening to a discharge passageway provided in the valve stem passes the diaphragm and the contents trapped within the transient metering chamber pass through the discharge passageway of the valve stem, exiting the stem outlet.

It will be appreciated that both the valve of a pressurized metered dose inhaler (pMDI) and also the pharmaceutical aerosol formulation each play an important part in obtaining optimum pharmaceutical performance from the product. In particular, pMDI valves represent a uniquely challenging application of metering valves, and generally need to meet many exacting requirements. For example the valve must be capable of adequately sealing pharmaceutical formulations based on pressurized, liquefied propellant systems, while minimizing any transmission of propellant out of the system and moisture into the system. Also the valve must be small and desirably inexpensive and must operate at suitably low actuation forces e.g. through reliable, smooth and easy movement of the valve stem. And of course upon actuation/operation of the valve stem, the valve must adequately sample and accurately meter the medicinal aerosol formulation.

The use of metal rather than plastic for valve stems and/or other internal valve components is frequently beneficial for minimizing distortion or material failure, e.g. of the valve stem upon use, as well as for avoiding many leachables from the plastic materials.

Metal valve stems are for example conventionally constructed by deep drawing or machining. Some shapes and configurations of valve stems may not be optimally manufactured using deep drawing, however. Additionally the thin-walled nature of deep drawn valve stems can leave large internal voids that can present problems such as drug deposition that can lead to complete occlusion of the valve stem. Machined metal valve components such as valve stems, e.g. formed using forging, turning and/or drilling, are relatively expensive. Machining is also disadvantageous in that the surface finish of the machined valve component can be quite rough, e.g. through the presence of machining marks. While extensive polishing can be used to improve the finish of external surfaces of such a component, unfavorably rough surfaces which cannot be polished, for example internal walls of passages and/or openings thereto in the valve stem, can provide seeding surfaces for drug deposition/accumulation, which in the case of a valve stem can lead to occlusion of its internal passages and/or openings.
Surprisingly, it has been found that metal powder injection molding (also known as metal injection molding) is particularly useful for the manufacture and provision of metal valve stems and/or valve bodies. The determined suitability of metal powder injection molding (referred to in the following as MPIM) is particularly surprising because in MPIM processes individual particles of a metal or a metal precursor are sintered to yield a metal component and although such processes would be expected to yield metal components having an unfavorable pebble-like surface finish and allowing for transmission of pressurized, liquefied propellant into and/or through said components, it has been found that MPIM can be used to provide valve components having desirable surface finishes, even without polishing, and desirable resistance to transmission of pressurized, liquefied propellant.
Accordingly the present invention proposes the use of MPIM for the manufacture of a metal valve component of a medicinal metered dose dispensing valve for use in a medicinal pressurized metered dose dispenser.

In particular the valve component is a valve stem that in its use in the pressurized metered dose dispenser is in contact with pressurized, liquefied propellant.
MPIM is also advantageous in that it allows for the manufacture of such valve components of various complex shapes and configurations to desirably tight tolerances and thus MPIM is particularly useful for the manufacture of valve stems. In regard to valve stems, MPIM is further advantageous in that it allows for the provision of internal passages and/or openings (e.g. side ports) thereto having desirable structural form and surface finish without a need for post-machining or finishing.
Valve components (e.g. valve stems and/or valve bodies) may be made of stainless steel, tool steel, high alloy steels or aluminum alloys. Valve components (e.g. valve stems and/or valve bodies) are preferably made of stainless steel. A variety of stainless steel grades can be worked by MPIM. In particular the use of MPIM allows for the provision of metal valve components made of stainless steel grades having higher corrosion resistance than the grades used in deep drawn or machined valve components. Thus another aspect is the provision of a valve stem made of 316, 316L-, 304-, 17-4PH-, 410- or 420-grade stainless steel. Desirably the valve component is made of a grade with high corrosion resistance, such as 304-, 17-4PH- 316 or 316L-grade stainless steel, more desirably 316 or 316L-grade stainless steel.
The present invention relates to a method of manufacturing a metal valve stem of a medicinal metered dose dispensing valve for use in a medicinal pressurized metered dose dispenser, the metal valve stem having a density of at least 98% of the bulk density of the metal, using metal powder injection molding, said method comprising the steps of
a) providing a mold for the valve component,
b) injecting into the mold a feedstock of metal particles and/or metal-containing precursor particles in a binder to provide a green part,
c) removing binder to provide a brown part, and
d) sintering the brown component.

The present invention may be used to manufacture a metered dose dispensing valve comprising a valve component described herein and a pressurized metered dose dispenser, e.g. a pressurized metered dose inhaler, comprising such a metered dose dispensing valve.

The dependent claims define further favorable embodiments.

The invention, its embodiments and further advantages will be described in the following with reference to the following drawings.
Figure 1 shows a cross-section through a metered dose dispensing valve.
Figure 2 shows a cross-section through an alternative metered dose dispensing valve.
Figure 3 shows a partial cross-section through a further metered dose dispensing valve.

It is to be understood that the present invention covers all combinations of particular and preferred aspects of the invention described herein.

MPIM (metal powder injection molding) is generally understood to include processes including injecting particles of metal (e.g. elemental metal and/or metal alloy) and/or metal-containing precursor (e.g. metal oxides or metal halides (e.g. metal chlorides)) in a binder into a mold cavity and further processing allowing for removal of binder and sintering of particles to provide a metal component. It is understood that MPIM does not include compression molding or isostatic pressing processes wherein a slab of metal material is squeezed by mold halves.

It has been found the MPIM is particularly useful for the manufacture of metal valve components (e.g. valve stems, valve bodies, such as primary valve bodies or secondary valve bodies) for metered dose dispensing valves for use in medicinal pressurized metered dose dispensers, such as pMDIs. This holds particularly true for valve components which in their use in such dispensers are in contact with pressurized, liquefied propellant and/or have complex geometries.
Metered dose dispensing valves for use in medicinal pressurized metered dose dispensers, such as pMDIs, typically comprise a valve stem co-axially slidable within a valve body (a primary valve body), an outer seal (e.g. diaphragm seal) and an inner seal (e.g. metering gasket). The outer and inner seals may be provided at the outer and inner ends of the valve body and the valve stem positioned in sliding sealing engagement with the seals so that a metering chamber is defined between the valve stem, valve body and seals. Alternatively the outer seal may be provided at the outer end of the valve body, the valve stem positioned in sliding sealing engagement with the outer seal, while the valve body, valve stem and inner seal are configured and positioned such that upon actuation of the valve, e.g. movement of the valve stem, the inner seal is operative to form a transient, fluid-tight seal between the valve stem and the valve body. In such valves a metering chamber, e.g. a transitory metering chamber, is favorably formed upon actuation. Metered dose dispensing valves generally comprise secondary valve bodies and depending on the particular design of the valve, such a secondary valve body can, for example, define a pre-metering region/chamber, a spring cage and/or a bottle emptier. Valve stems, primary valve bodies and/or secondary valve bodies of such valves are favorably manufactured via MPIM.

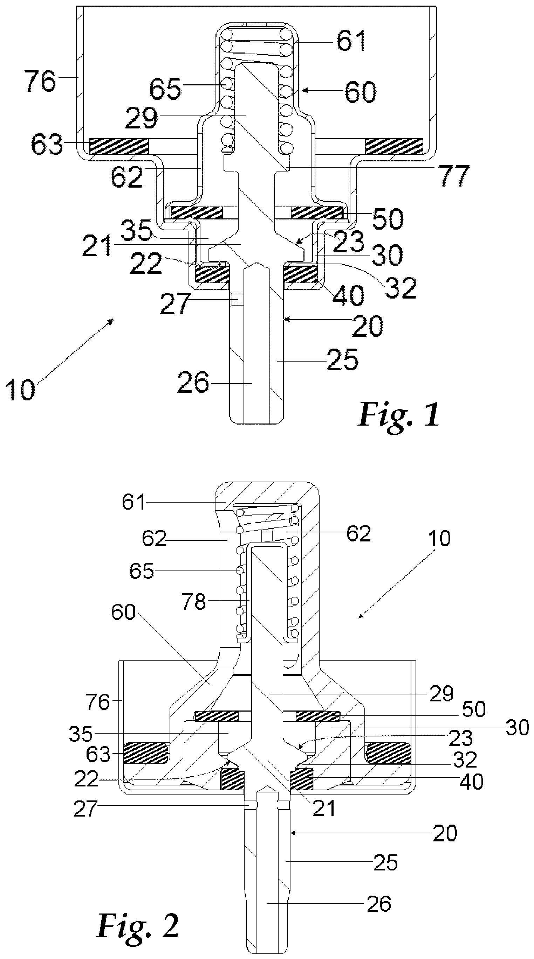

Figures 1-3 illustrate examples of metered dose dispensing valves which favorably include valve components manufactured via MPIM.
Figures 1 and 2 illustrate two embodiments of a metered dose dispensing valve (10) of the general type described in WO 2004/022142. In use, such valves are crimped onto an aerosol container (not shown) using a ferrule (76) where a gasket seal (63) is located between the valve ferrule and the opening of the aerosol container to ensure a gas-tight seal between the valve and the aerosol container. Formulation within the aerosol container will surround and contact inner components of the valve. Referring to Figures 1 and 2, in each exemplary embodiment, the valve comprises a valve stem (20) that generally defines a longitudinal axis and comprises a body portion (21) and a stem portion (25) including a discharge passageway (26), and a valve body (30), where an internal chamber (35) is defined at least in part by at least a portion of the inner surface of the valve body. The body portion (21) of the valve stem is generally positioned within a portion of the internal chamber (35). As can be recognized from Figures 1 and 2, the body portion of the valve may be generally triangular or diamond-shaped in its cross section. Moreover in such valves, the body portion of the valve stem favorably comprises a metering surface (22) near the stem portion (25) of the valve stem, wherein the longitudinal axis and a plane tangential to at least a portion of the metering surface define an angle from about 2° to about 90°. The embodiment shown in Figure 1 has an angle of about 90°, while the embodiment shown in Figure 2 has an angle of about 55°. Also as can be appreciated from Figures 1 and 2, the valve body (30) comprises a metering portion (32) the surface of which is configured to substantially conform to the metering surface of the valve stem. Each valve includes a diaphragm seal (40) having walls that define an aperture in slidable, sealing engagement with the stem portion (25) of the valve stem, and a metering gasket (50). The metering gasket (50) is configured and positioned such that upon actuation of the valve (e.g. movement of the valve stem inwardly) a transient, substantially fluid-tight face seal can be formed between the metering gasket and the valve stem (20), in particular between the body portion (21) of the valve stem, more particular a sealing surface (23) of the body portion. As will be appreciated by the skilled reader, upon movement of the valve stem inwardly a (transitory) metering chamber (not visible) is formed between the metering surface (22) of the valve stem and the metering portion (32) of the valve body and upon formation of the described face seal, aerosol formulation in the thus-formed metering chamber is isolated from the aerosol container. Upon further movement of the valve stem inwardly, an opening (27) into the discharge passageway (26) of the valve stem passes the diaphragm seal and the contents of the metering chamber pass through the discharge passageway of the valve stem, exiting the stem outlet. As can be appreciated from the embodiments shown in Figures 1 and 2, in such valves the sealing surface (23) of the body portion (21) of valve stem (20) is desirably generally conical or conical. Moreover it is favorable that the longitudinal axis and a plane tangential to at least a portion of the sealing surface define an angle from about 30° to about 80°. Referring to Figures 1 and 2, in each exemplary embodiment, the valve typically comprises a second valve body (60) defining a spring cage (61) for holding a compression spring (65). One end of the compression spring abuts the inner, upper wall of the spring cage and the other end abuts either a flange (77) on an upper stem portion (29) of the valve stem (see Figure 1) or a separate flange component (78) mounted onto the upper stem portion of the valve stem (see Figure 2). One or more inlets (62) typically traversing the spring cage provide open and substantially unrestricted fluid communication between the interior chamber (35) and the aerosol container (not shown). The valve stem (20), primary valve body (30) and/or secondary valve body (60) of such valves are favorably manufactured via MPIM.

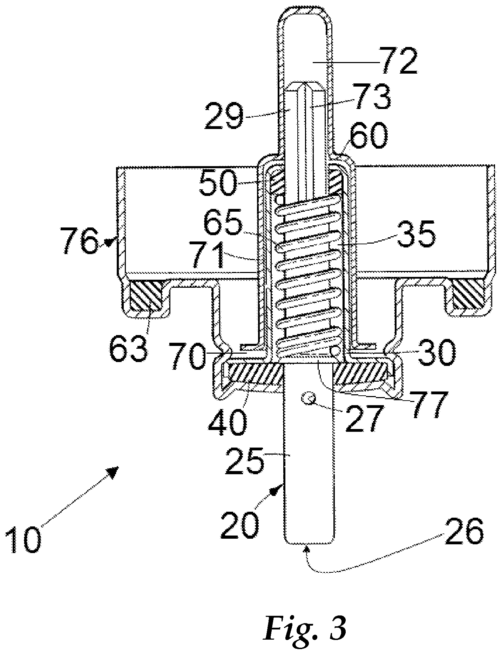

Figure 3 provides a partial cross-sectional view of another exemplary metered dose dispensing valve (10). Similar to the valves shown in Figures 1 and 2, in use, the valve is crimped onto an aerosol container (not shown) via a ferrule (76), and a gasket seal (63) is provided to ensure a gas tight seal. Referring to Figure 3, the valve (10) comprises a valve stem (20) that generally defines a longitudinal axis and a valve body (30), where the valve stem extends through a central aperture of the valve body. A lower stem portion (25) of the valve stem extends outwardly and is in slidable, sealing engagement with a diaphragm seal (40), while an upper stem portion (29) of the valve stem extends inwardly and is in slidable, sealing engagement with a metering gasket (50). A (non-transistory) metering chamber (35) is defined within the valve body (30) between the diaphragm seal (40) and metering gasket (50). A compression spring (65) is positioned within the valve body with one end abutting the metering gasket (50) and the other end abutting a flange (77) on the valve stem near the diaphragm seal. As will be appreciated by the skilled reader, upon movement of the valve stem inwardly, a groove (73) in the upper stem portion (29) will pass beyond the metering gasket (50) so that a complete seal is formed between the upper stem portion of the valve stem and the metering gasket, thereby sealing off the metering chamber. Upon further movement of the valve stem inwardly, an opening (27) into a discharge passageway (26, not visible since the valve stem is not shown in cross section) of the valve stem passes the diaphragm seal into the metering chamber and the contents of the metering chamber pass through the discharge passageway of the valve stem, exiting the stem outlet. Referring to Figure 3, the valve may comprise a second valve body (60) defining a bottle emptier. When such a secondary valve body is provided, aerosol formulation in the aerosol container (not shown) will pass through a gap (70) between the first and second valve bodies (30 and 60) (the gap is near the diaphragm seal), through an annular gap (71) into a pre-metering region (72) and then through the groove (73) into the metering chamber (35). The valve stem (20), primary valve body (30) and/or secondary valve body (60) of such valves are favorably manufactured via MPIM.

Metal particles and/or metal-containing precursor particles (hereinafter generally referred to as particles) used in the MPIM feedstock are favorably selected such that the manufactured valve component is made of stainless steel, tool steel, high alloy steel or aluminum alloy. Preferably, particles are selected such that the manufactured valve component is made of stainless steel. It has been found that through the use of MPIM, stainless steel metal valve components can be made of grades of stainless steel with higher corrosion resistance than previously possible using deep drawing and machining fabrication routes. Accordingly the particles are advantageously selected such that the manufactured valve component is made of a grade of stainless steel selected from the group consisting of 316-grade, 316L-grade, 304-grade, 17-4PH-grade, 410-grade and 420-grade stainless steel, more particularly a grade of stainless steel selected from the group consisting of 316-grade, 316L-grade, 304-grade and 17-4PH-grade stainless steel, most particularly 316-grade or 316L-grade stainless steel.

A small particle size is desirable as it tends to lead to a better (smoother) surface texture (e.g. roughness on a scale of generally less than 3 microns and more favorably less than 1 micron) on the finished component and very low porosity in the final component. Finer particles also allow for faster de-binding and sintering processes, with consequent financial benefits. Preferably the median particle size is about 25 microns or less, more preferably 20 microns or less, and most preferably about 15 microns or less.

It is desirable that the particles have a distribution of controlled and uniform particle sizes. A narrow size distribution range is generally considered preferable as it helps to reduce the tendency for particle segregation at any stage in the handling and molding process, thereby helping to produce more consistent final parts, with even and consistent shrinkage and better dimensional control. Although a broader range of particle sizes can be used in order to improve particle packing density, it has been found that, if a narrow size range is used, particle loading and sintering conditions can be optimized to increase the density of the final part. Preferably, the particles have a size distribution that is 80% or more (by mass) in the size range of about 25 microns or less, more preferably in the range of about 20 microns or less and most preferably in the range of about 15 microns or less. In terms of the aforesaid ranges, it is favorable that the particles have a size distribution that is 80% or more (by mass) in the size range of about 0.5 microns or more, more favorably in the range of about 5 microns or more and most favorably in the range of about 7 microns or more.

Desirably particles are spherical and/or approximately spherical in shape. Spherical and/or approximately spherical particles tend to prevent particle alignment issues in the final molded parts, leading to better quality, surface finish and dimensional consistency.

Particles can either be provided in the form of particles which have the desired final alloy composition (e.g. by gas atomization of the molten alloy), or can be provided as a mixture of particles of differing compositions which can be blended to give the desired final composition after sintering. For example, iron particles can be blended with alloying additive particles (e.g. vanadium, manganese), and the final steel composition can then be obtained by sintering. This latter approach does require the ability to produce a uniform and un-segregated powder blend, however, and relies on solid state diffusion to produce the desired final alloy composition. This can be difficult, particularly where the particles are not very small. Preferably, the former approach is used, where the metal particles themselves each have the composition of the desired alloy. In particular, it is desirable to use metal particles having an alloy composition corresponding to a grade of stainless steel selected from the group consisting of 316-grade, 316L-grade, 304-grade, 17-4PH-grade, 410-grade and 420-grade stainless steel.

An alternative approach to the use of elemental metal or metal alloy particles is to use an appropriate mix of metal-containing precursor material particles, reduced or otherwise reacted to form the metal or metal alloy either before or after molding. This chemical transformation can take place as part of the solid-state sintering process. For example, a mixture of metal oxide particles may be used, reduced to the corresponding elemental metals during the heating process, for example as described in US patent 6,849,229.

It is preferred to use in the feedstock particles of metal, e.g. elemental metal particles and/or metal alloy particles.

Particles for MPIM are widely commercially available, e.g. from BASF, QMP (Rio Tinto Group), Daido Steel and Höganäs.

Also a variety of different processes can be used to prepare particles appropriate for MPIM. Examples of such processes include gas-atomization of molten metal, water-atomization, mechanical milling or grinding, or the carbonyl iron deposition process. The gas-atomization process, in which molten metal (elemental metal or metal alloy) is sprayed from a nozzle in the form of small droplets, is particularly suitable in providing spherical metal particles. Modifiers (e.g. 0.1-1% Si) can be added to the melt in order to improve nozzle flow and produce finer atomization (smaller particle sizes), however it is desirable to keep the level of silicon added as low as possible. Another process that is particularly suitable for the production of fine spherical metal particles is the carbonyl iron process, in which the chemical decomposition of metal carbonyls is used to form metal powders, such as iron powder produced by the decomposition of iron pentacarbonyl. Similarly nickel powder can be produced from the decomposition of nickel tetracarbonyl, chromium powder can be produced from the decomposition of chromium hexacarbonyl, etc. This process has the disadvantage compared to gas atomization that metal carbonyls are poisonous. In addition, the decomposition process requires quite a high energy input and is quite expensive. Also, each individual particle is elemental, rather than in the form of an alloy. In general, for control of particle size and shape, the gas-atomization process is preferred. Alternative powder production processes, which are generally less preferred because the particles produced tend to be more irregular and less spherical, include: (1) water-atomization to produce metal alloy powder; (2) reduction from iron oxide powder followed by grinding/milling (e.g. in an inert atmosphere).

Particles are blended in a binder to form a feedstock to be used for injection molding.

The principle function of a binder is to (initially) bind the particles (e.g. holding a molded green-part together when the part is removed from the mold) and provide lubrication. A variety of different binders, e.g. binder systems, can be used in MPIM. Typically multicomponent binders are used, including a variety of different components performing different functions. Typical feedstocks comprise 7-40% (by weight) of a binder (e.g. a binder system) with the balance being the metal particles and/or metal-containing precursor particles. It is favorable to have the metal particle and/or metal-containing precursor particle loading as high as is practical, in order to minimize part shrinkage and deformation during sintering and densification; preferably at least about 70% by weight particle loading, more preferably at least 80%.

As mentioned above, typically a binder comprises multiple components. In particular it has been found useful to provide a binder comprising a plurality of components having different melting points. The lowest melting point component can thus be removed first, as the part is subsequently heated up to de-binder it, followed by higher melting temperature components. Preferably, a single high melting point component may be left to hold the "brown" part together until the start of the sintering stage. As an alternative to a binder comprising meltable binder components, a binder comprising a plurality of components having different thermal decomposition points may be used.

Typical binders are based on polyolefin thermoplastic materials, such as low molecular weight polyethylene or polypropylene. Other suitable binders include systems based on polystyrene, polyvinylchloride, polyethylene carbonate, or polyethylene glycol. Preferably, a binder comprising a polyolefin is used. More preferably, a binder comprising two or more polyolefins and/or polyolefin waxes having different melting points is used.

Alternatively, binders may be suitably water-based. For example, a water-based agar or a water-soluble component based on cellulose can be used as a binder. For example, the water based agars can form a gel network that binds the particles together.

A binder also typically includes a component to act as a lubricant, to facilitate the flowability of the feedstock when heated, allowing the feedstock to be injected into the mold. Suitable lubricants include a low melting point wax. Examples include paraffin wax, microcrystalline wax, Carnauba wax, and water-soluble polymers. For wax based lubricant systems, multiple waxes may be used together to make up the lubricant.

A combination of two or more polyolefins with a wax is one of the most commonly and suitably used binders.

Additional components of a binder can include resins, plasticisers and surfactants. Other additives may also be used. These can include for example elasticisers, antioxidants, and organometallic coupling agents. For example, about 1% of stearic acid can be added to act as a surfactant and a mold release agent. The selection of additional components can readily be made by those skilled in the art, based on the size of the desired parts, their required dimensional tolerances and surface finish characteristics, the acceptable cost limits, etc.

The preparation of the feedstock, e.g. addition of components of a binder to metal particles and/or metal-containing precursor particles, can be carried out in multiple ways. These include both dry processes (e.g. dry blending, dry milling, or fluidization techniques) and wet processes (e.g. wet milling or slurry mixing). Different individual components might be added by different techniques. For example, spray coating can be used to apply liquid components (e.g. surfactants) to the powder particles, etc. Mixing may take place in an inert atmosphere where desired. The most appropriate blending or compounding approaches will depend on the constituents to be mixed to make the chosen feedstock, and such techniques are known to those skilled in the art of MPIM, where the prime objective is to ensure adequate homogeneity of the feedstock. For example for a feedstock including a binder based on thermoplastic polymers (e.g. polyolefins) plus a wax, a preferred approach is to pass blended metal particles into a heated kneader/mixer system, where the molten wax components are added and kneaded in, followed similarly by molten thermoplastic polymers. The feedstock thus mixed is then cooled and mechanically granulated into desirably uniform granules/pellets of a few millimeters across.

Once the feedstock is prepared, the procedure of injection into the mold in MPIM is similar to the injection of feedstock in plastic injection molding, and MPIM allows the prepared feedstock to be readily injection molded into very many different configurations, including small and intricate features. The molding presses used (e.g. with heated screw-feed injection systems) and the molds used (e.g. hot runner multi-cavity molds) are also similar to those used for standard plastic injection molding. The same rules of part and mold design also apply, with respect to such considerations as wall thicknesses and their consistency, draft angles, tooling parting lines, gas vents, injection gates, ejector pins, undercuts, shut-outs, etc. The principal difference is that MPIM molds need to have oversize dimensions to allow for the shrinkage that occurs during sintering, so that the final part is to the required dimensions. Shrinkage rates are predictable and are well understood by those skilled in the art, so final tolerances can be reasonably well controlled.

Temperatures used during injection of the feedstock into the mold are those appropriate allowing for adequate melt flow of the particular binder system, typically in the range from about 180 to about 300°C.

Typically after injection, the part is held briefly in the mold until it has cooled enough to eject. Cooling channels and coolant circulation in the mold may be used to accelerate this process, in order to reduce cycle times. Ejection from the mold is entirely analogous to that employed in standard plastic injection molding. Parts may be released entirely by gravity, but preferably suitably placed ejector pins are used to strip parts from the molding tool cavity.

If desired a green part could be machined at this stage, if any machining operations were desired and/or needed. This is called "soft machining". Generally parts for valve components (e.g. valve stems and/or valve bodies) do not require any such operations.

As mentioned above there are various different binders, and differing de-binding processes are typically applied for differing binders. The principle methods for de-binding include thermal, solvent and catalytic de-binding processes. The prepared green part may or may not need support at this stage.

Thermal de-binding processes can be conducted in either a static furnace with a temperature/time profile control system, or in a continuous conveyor belt furnace. Often, a de-binding process is arranged to lead directly into the sintering process. Because of the long overall cycle times typically involved (often many hours) in de-binding and sintering, static furnaces can be more cost-effective for most applications.

Specific details of a particular thermal de-binding process depend on the nature and composition of the binder used. For example, for a binder comprising one or more waxes and organic polymer components, such as polyolefins, the following general de-binding process can be used. Typically the wax or waxes are first removed by gradually heating the green part to a temperature from about 80°C to about 120°C. Generally a heating rate is applied which does not exceeding 300°C per hour. Temperature holds may be employed at temperatures at which different wax components of the binder will melt and run out of the green part. Green parts may be placed on a bed of alumina powder to facilitate removal of wax components, e.g. to pull the molten wax(es) out by capillary action. Removal of wax(es) leaves very fine channels through the material of the part, through which other components of the binder (e.g. polymeric components) can subsequently pass upon their removal. Once sufficient time has been allowed for the wax component(s) to be melted out of the part, the temperature can be raised further by controlled heating to temperatures at which the organic polymer components are volatilized. Typically, this involves ramping the temperature up to between about 300°C and about 400°C, although depending on the particular components of the binder, temperatures of 600°C or more can be used. The heating rate typically does not exceed 100°C per hour. Similar to the process used in the removal of wax components, multiple holds at different temperatures may be employed, holding at temperatures corresponding to the volatilization or thermal depolymerization (pyrolysis) temperatures of the different constituents of the binder.

A variety of different atmospheres may be used during thermal de-binding processes. Air, vacuum (e.g. < 10 mbar), inert gas (e.g. argon), or a reducing atmosphere (e.g. dry hydrogen or Naton (10% hydrogen, 90% nitrogen)) may be used. Post-treatments at higher temperatures in reducing atmospheres (e.g. 1 hour in hydrogen at reduced pressure at 1000°C) may be used if metal oxidation has occurred.

As is well known to those skilled in the art, thermal cycles are typically designed to avoid distortion or damage to the initial green part and the resulting brown part after de-binding. For example, in general, rapid heating to elevated temperatures is avoided, so that volatile components of the binder do not out-gas at rates greater than the gas can leak or diffuse out. If a water-based binder system is used, often a pre-drying step (e.g. a slow heating to 110°C) is employed. As another example, typically the rate of generation of liquid melt during de-binding is kept low because if too much of the binder, for example wax component(s), is melted at the same time, the resultant liquid flow can lead to slumping of the parts. Depending on component size and/or binder composition, particular cycles of gradual and staged temperature rises are chosen such as to avoid excess thermal stresses or softening or melting of the parts, while allowing for a reasonable de-binding rate.

Solvent de-binding processes may be used alone or in combination with other methods. For example, much of the binder may be washed out or extracted with a solvent, leaving behind a thermosetting component that can be hardened by exposure to ultraviolet radiation. Alternatively, most of the binder may be flushed out with a solvent (or a vapor), leaving residual binder and, if applicable, residual solvent to be removed by thermal methods. Mineral spirits and water are examples of solvents used to remove binder components. Similar to thermal de-binding processes the skilled person understands that for solvent de-binding the particular process used is chosen so as to avoid risk of part distortion while allowing for a reasonable de-binding rate.

Catalytic de-binding processes typically involve the addition of a catalyst into the binder. In such processes the added catalyst facilitates the break up of molecules, e.g. polymeric molecules, of binder components into smaller molecules having relatively high vapor pressures which can then be removed at relatively low temperatures. For example, acid catalysts can be used to break up polyacetal binder components into formaldehyde.

Upon de-binding of a green part, a brown part is provided. Such brown parts typically have little strength and are very fragile until sintering together of the particles takes place. Typically, brown parts are primarily held together by a small amount of residual binder that is finally removed during the subsequent sintering process, e.g. by decomposition at or around the higher temperatures used for sintering.

During sintering, brown parts are heated to temperatures high enough to cause the particles to bond together in the shape of the desired part. This bonding is generally a solid state fusion process. The bonding occurs at temperatures below the melting point of the metal e.g. elemental metal and/or metal alloy. For particles comprising metal precursor materials, such as metal oxides, bonding occurs as a result of their thermal decomposition (reduction) to the corresponding elemental metals, which then fuse together in the solid state.

Typical sintering temperatures are 1200 - 1450°C. Suitable selection of sintering heating rates and holding times are well known to those skilled in the art. For example heating is typically performed at rates that prevent excessive distortion of the part, while sintering times are typically long enough to allow for any required solid state diffusion to occur. For metal valve components, such as valve stems and/or valve bodies, made of 316 or 316L grade stainless steel, sintering is generally carried out at approximately 1400°C or more in order to provide adequate sintering and adequate strength and density. Static or continuous sintering furnaces can be used; the latter are common. Sintering may be performed in an inert or reducing atmosphere.

By the end of the sintering process, part shrinkage will be complete and can often reach around 20%. Typically shrinkage is on the order of about 15 to about 20%. Surprisingly for a particular feedstock and a desired metal valve component form, shrinkage can be predicted and/or controlled so well that metal valve components (in particular valve stems and/or valve bodies) can be produced that meet the stringent dimensional tolerances of these very demanding applications.

Sintering leads to the elimination of any residual components of the binder and to densification and strengthening of the part. Final densities of the sintered part are desirable at least 98% or more, or even more desirably at least 99% or more, of the bulk density of the metal. What little porosity remains is generally closed-cell so that any propellant leakage through MPIM-manufactured valve components during use in a pressurized medicinal dispensing device (e.g. a pMDI) is prevented.

After sintering, post-MPIM operations may be carried out on the resulting metal part as desired and/or needed. For example surface polishing may be performed. However it has been surprisingly found that typically no post-MPIM operations, such as polishing or machining, are needed Moreover it has been advantageously found that the resulting metal part obtained after sintering can be used directly as a metal valve component without any further processing. Thus MPIM processes as described herein allow for the ready and inexpensive manufacture of metal valve components (e.g. valve stems and/or valve bodies) for medicinal metered dose dispensing valves and pressurized metered dose dispensers (e.g. pMDIs) without the need for expensive metal machining and/or polishing.

It has been found that metered dose dispensers including valves as shown in Figure 3 with valve stems made of 316-grade stainless steel by MPIM as described herein show favorable valve operation with reliable, smooth and easy movement with desirably low actuation forces and thus favorable friction characteristics. Such dispensers filled with a formulation containing 8% w/w ethanol in HFA 134a had a leak rate of less than 100 mg per year when stored for 7 days at 30 degrees C. Dose weights of 59 mg dispensed from such dispensers had a standard deviation of less than 1 mg. Examination of the valve stems by Scanning Electron Microscopy revealed that the surface finish was surprisingly smooth with a roughness less than 1 micron.

## Claims

1. A method of manufacturing a metal valve stem of a medicinal metered dose dispensing valve for use in a medicinal pressurized metered dose dispenser, the metal valve stem having a density of at least 98% of the bulk density of the metal, using metal powder injection molding, said method comprising the steps of:
a) providing a mold for the valve stem,
b) injecting into the mold a feedstock of metal particles and/or metal-containing precursor particles in a binder to provide a green part;
c) removing binder to provide a brown part; and
e) sintering the brown part.

2. A method according to claim 1 in which the metal particles and/or metal-containing precursor particles are selected such that the manufactured valve component is made of stainless steel, tool steel, high alloy steel or aluminum alloy.

3. A method according to claim 2 in which the metal particles and/or metal-containing precursor particles are selected such that the manufactured valve component is made of stainless steel, said stainless steel being a grade of stainless steel selected from the group consisting of 316-grade, 316L-grade, 304-grade, 17-4PH-grade, 410-grade and 420-grade stainless steel.

4. A method according to any one of the preceding claims in which the median particle size of the metal particles and/or metal-containing precursor particles of the feedstock is about 25 microns or less.

5. A method according to any one of the preceding claims in which the particle size distribution of the metal particles and/or metal-containing precursor particles of the feedstock is 80% or more by mass in the size range of about 25 microns or less.

6. A method according to claim 5 in which the particle size distribution of the metal particles and/or metal-containing precursor particles of the feedstock is 80% or more by mass in the size range of about 0.5 microns or more.

7. A method according to any one of the preceding claims in which the metal particles and/or metal-containing precursor particles of the feedstock are spherical and/or substantially spherical.

8. A method according to any one of the preceding claims in which the feedstock comprises 60% or more by mass of metal particles and/or metal-containing precursor particles.

## Patentansprüche

1. Verfahren zum Herstellen eines Metallventilschafts eines medizinischen Dosierabgabeventils zur Verwendung in einer unter Druck stehenden medizinischen Dosierabgabevorrichtung, wobei der Metallventilschaft eine Dichte von mindestens 98 % der Massendichte des Metalls aufweist, unter Verwendung von Metallpulver-Spritzgießen, wobei das Verfahren die folgenden Schritte aufweist:
a) Bereitstellen einer Form für den Ventilschaft,
b) Einspritzen eines Rohstoffs aus Metallteilchen und/oder metallhaltigen Vorläuferteilchen in einem Bindemittel in die Form, um einen Grünkörper bereitzustellen;
c) Entfernen von Bindemittel, um einen Braunkörper bereitzustellen; und
d) Sintern des Braunkörpers.

2. Verfahren nach Anspruch 1, bei dem die Metallteilchen und/oder metallhaltigen Vorläuferteilchen so ausgewählt werden, dass der hergestellte Ventilbestandteil aus Edelstahl, Werkzeugstahl, hochlegiertem Stahl oder Aluminiumlegierung hergestellt ist.

3. Verfahren nach Anspruch 2, bei dem die Metallteilchen und/oder metallhaltigen Vorläuferteilchen so ausgewählt sind, dass der hergestellte Ventilbestandteil aus Edelstahl hergestellt ist, wobei der Edelstahl ein Grad von Edelstahl ist, der ausgewählt ist aus der Gruppe bestehend aus Edelstahl des Grades 316, des Grades 316L, des Grades 304, des Grades 17-4PH, des Grades 410 und des Grades 420.

4. Verfahren nach einem der vorstehenden Ansprüche, bei dem die mittlere Teilchengröße der Metallteilchen und/oder metallhaltigen Vorläuferteilchen des Rohstoffs etwa 25 Mikrometer oder weniger beträgt.

5. Verfahren nach einem der vorstehenden Ansprüche, bei dem die Teilchengrößenverteilung der Metallteilchen und/oder metallhaltigen Vorläuferteilchen des Rohstoffs 80 Masse-% oder mehr in der Größenordnung von etwa 25 Mikrometer oder weniger beträgt.

6. Verfahren nach Anspruch 5, bei dem die Teilchengrößenverteilung der Metallteilchen und/oder metallhaltigen Vorläuferteilchen des Ausgangsmaterials 80 Masse-% oder mehr in der Größenordnung von etwa 0,5 Mikrometer oder mehr beträgt.

7. Verfahren nach einem der vorstehenden Ansprüche, bei dem die Metallteilchen und/oder metallhaltigen Vorläuferteilchen des Rohstoffs kugelförmig und/oder im Wesentlichen kugelförmig sind.

8. Verfahren nach einem der vorstehenden Ansprüche, bei dem der Rohstoff 60 Masse-% oder mehr Metallteilchen und/oder metallhaltige Vorläuferteilchen aufweist.

## Revendications

1. Procédé de fabrication d'une tige de soupape en métal d'une soupape de distribution de dose médicinale mesurée pour une utilisation dans un distributeur-doseur médicinal sous pression, la tige de soupape en métal ayant une masse volumique d'au moins 98 % de la masse volumique en vrac du métal, en utilisant un moulage par injection de poudre métallique, ledit procédé comprenant les étapes consistant à :
a) fournir un moule pour la tige de soupape,
b) injecter dans le moule un produit de départ de particules de métal et/ou de particules de précurseur contenant du métal dans un liant pour fournir une pièce crue ;
c) éliminer le liant pour fournir une pièce brune ; et
d) fritter la pièce brune.

2. Procédé selon la revendication 1, dans lequel les particules de métal et/ou les particules de précurseur contenant du métal sont choisies de telle sorte que le composant de soupape fabriqué est constitué d'acier inoxydable, d'acier à outil, d'acier fortement allié ou d'alliage d'aluminium.

3. Procédé selon la revendication 2, dans lequel les particules de métal et/ou les particules de précurseur contenant du métal sont choisies de telle sorte que le composant de soupape fabriqué est constitué d'acier inoxydable, ledit acier inoxydable étant un grade d'acier inoxydable choisi dans le groupe constitué d'acier inoxydable de grade 316, de grade 316L, de grade 304, de grade 17-4PH, de grade 410, et de grade 420.

4. Procédé selon l'une quelconque des revendications précédentes, dans lequel la taille médiane de particules des particules de métal et/ou des particules de précurseur contenant du métal du produit de départ est d'environ 25 micromètres ou moins.

5. Procédé selon l'une quelconque des revendications précédentes, dans lequel la distribution granulométrique des particules de métal et/ou des particules de précurseur contenant du métal du produit de départ vaut 80 % ou plus en masse dans la plage granulométrique d'environ 25 micromètres ou moins.

6. Procédé selon la revendication 5, dans lequel la distribution granulométrique des particules de métal et/ou des particules de précurseur contenant du métal du produit de départ vaut 80 % ou plus en masse dans la plage granulométrique d'environ 0,5 micromètre ou plus.

7. Procédé selon l'une quelconque des revendications précédentes, dans lequel les particules de métal et/ou les particules de précurseur contenant du métal du produit de départ sont sphériques et/ou sensiblement sphériques.

8. Procédé selon l'une quelconque des revendications précédentes, dans lequel le produit de départ comprend 60 % ou plus en masse de particules de métal et/ou de particules de précurseur contenant du métal.
